(19) Europäisches Patentamt
European Patent Office
Office européen des brevets

(11) Veröffentlichungsnummer: **0 209 773**
**B1**

# (12) EUROPÄISCHE PATENTSCHRIFT

(45) Veröffentlichungstag der Patentschrift: **04.10.89**

(51) Int. Cl.⁴: **C07D 495/14**, C07D 409/14 // (C07D495/14, 333:00, 333:00, 223:00)

(21) Anmeldenummer: **86109186.6**

(22) Anmeldetag: **04.07.86**

(54) **Verfahren zur Herstellung von 4,5-Dihydro-dithieno[3,4b:3',4'-e]-azepin-5,9-dion.**

(30) Priorität: **11.07.85 DE 3524743**

(43) Veröffentlichungstag der Anmeldung: **28.01.87 Patentblatt 87/5**

(45) Bekanntmachung des Hinweises auf die Patenterteilung: **04.10.89 Patentblatt 89/40**

(84) Benannte Vertragsstaaten: **AT BE CH DE FR GB IT LI NL SE**

(56) Entgegenhaltungen:
**DE-A- 3 037 971**
**DE-A- 3 309 719**
**GB-A- 1 197 692**

(73) Patentinhaber: **BASF Aktiengesellschaft, Carl-Bosch-Strasse 38, D-6700 Ludwigshafen(DE)**

(72) Erfinder: **Steiner, Gerd, Dr., Oberer Waldweg 1, D-6719 Kirchheim(DE)**
Erfinder: **Thyes, Marco, Dr., Thorwaldsenstrasse 1, D-6700 Ludwigshafen(DE)**

**EP 0 209 773 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von 4,5-Dihydro-dithieno[3,4-b:3',4'-e]-azepin-5,9-dion.

Die bislang bekannten Verfahren zur Herstellung von 4,5-Dihydro-dithieno[3,4-b:3',4'-e]azepin-5,9-dion (vgl. DE-OS 3 037 971 in Verbindung mit DE-OS 3 309 719) sind langwierig und verlaufen besonders bei größeren Ansätzen mit recht mäßigen Ausbeuten.

Es wurde nun gefunden, daß man das 4,5-Dihydro-dithieno[3,4-b:3',4'-e]azepin-5,9-dion der Formel I

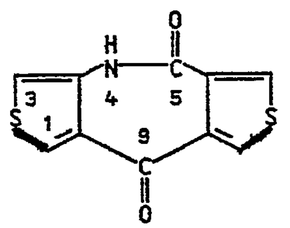

I

sehr gut erhalten kann, indem man Thiophen-3,4-dicarbonsäure-2,5-dichlorthien-3-yl-amid der Formel II

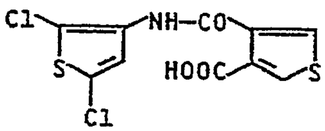

II

durch Behandlung mit N-Hydroxysuccinimid in Gegenwart eines Mol-äquivalents eines Kondensationsmittels in den Ester der Formel IV

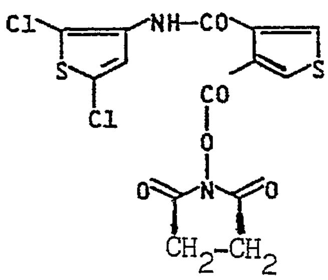

IV

überführt, diesen nach Friedel-Crafts cyclisiert und anschließend das so erhaltene 1,3-Dichlor-4,5-dihydro-dithieno[3,4-b:3',4'-e]azepin-5,9-dion der Formel III

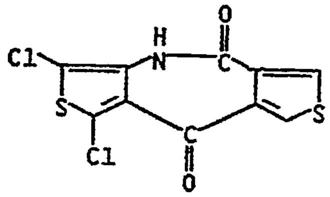

III

katalytisch entchloriert.

Die Umsetzung II→IV geschieht zweckmäßig in einem inerten organischen Lösungsmittel, wie einem Kohlenwaserstoff, z.B. Toluol, oder insbesondere einem gesättigten aliphatischen oder gesättigten cyclischen Ether, wie Diethylether, Tetrahydrofuran oder Dioxan, oder in einem dipolaren aprotischen Lösungsmittel, wie Dimethylformamid. Die Reaktion ist in der Regel bei Raumtemperatur in etwa 1 bis 3 Stunden beendet. Der ausgefallene Harnstoff wird abgesaugt oder durch Extraktion abgetrennt und der aktivierte Ester IV durch Einengen isoliert.

Der so erhaltene Ester IV wird anschließend nach Friedel-Crafts cyclisiert. Hierzu setzt man den aktivierten Ester IV in Gegenwart eines 5- bis 12fachen Überschusses Aluminiumchlorid in einem inerten aprotischen organischen Lösungsmittel, insbesondere einem gesättigten aliphatischen oder gesättigten cyclischen Ether, wie Diethylether, Tetrahydrofuran oder Dioxan, oder vorzugsweise in einem dipolaren

aprotischen Lösungsmittel wie Dimethylformamid bei Temperaturen zwischen 50 und 120°C während 0,5 bis 2 Stunden zum 1,3-Dichlor-4,5-dihydro-dithieno[3,4-b:3',4'-e]azepin-5,9-dion in glatter Reaktion um.

Diese Reaktionsfolge muß beibehalten werden, da normale Friedel-Crafts-Cyclisierungen über das Säurechlorid oder andere aktivierte Derivate nicht zum Siebenring, sondern ausschließlich zum entsprechenden Fünfrin-Imid führen.

Das 1,3-Dichlor-4,5-dihydro-dithieno[3,4-b:3',4'-e]azepin-5,9-dion III wird entchloriert durch katalytische Hydrierung unter Verwendung von Edelmetallkatalysatoren, wie z.B. Palladium auf Kohlenstoff, in Gegenwart eines Säurefängers, wie z.B. Natriumacetat, Natriumcarbonat, Ammoniak oder eines Alkylamins, wie z.B. Tripropylamin, in einem inerten organischen Lösungsmittel, insbesondere einem gesättigten aliphatischen oder gesättigten cyclischen Ether, wie Diethylether. Tetrahydrofuran oder Dioxan, einem dipolaren aprotischen Lösungsmittel, vorzugsweise Dimethylformamid oder 1-Methyl-pyrrolidon-2 oder einem niederen Alkohol, vorzugsweise Propanol oder Isopropanol. Es wird bei einem Wasserstoffdruck zwischen 1 und 3 Atmosphären bei Temperaturen zwischen Raumtemperatur und 100°C während 2 und 8 Stunden in das 4,5-Dihydro-dithieno-[3,4-b:3',4'-e]azepin-5,9-dion der Formel I übergeführt. Gegebenenfalls kann bei Verwendung eines Wasserstoffdonors, wie z.B. Ammoniumformiat, auf den Einsatz von elementarem Wasserstoff verzichtet werden.

Das Thiophen-3,4-dicarbonsäure-2',5'-dichlor-thien-3-yl-amid der Formel II, welches als Ausgangsmaterial dient, erhält man in einfacher Weise durch Umsetzung von Thiophen-3,4-dicarbonsäureanhydrid mit 2,5-Dichlor-3-amino-thiophen in einem inerten organischen Lösungsmittel, wie einem Kohlenwasserstoff, vorzungsweise Toluol, einem Halogenkohlenwasserstoff oder einem gesättigten aliphatischen oder gesättigten cyclischen Ether, wie Diethylether, Tetrahydrofuran oder Dioxan, bei Raumtemperatur während 1 bis 5 Stunden.

Das neue Verfahren zeichnet sich dadurch aus, daß man das 4,5-Dihydro-dithieno [3,4-b:3',4'-e]azepin-5,9-dion aus leicht zugänglichen Ausgangsmaterialien in sehr guter Ausbeute erhält. Darüber hinaus kann es in dengroßtechnischen Maßstab übertragen werden, ohne daß die Ausbeute absinkt.

Die folgenden Beispiele dienen der Erläuterung der Erfindung.

<u>Beispiel 1</u>

a) Thiophen-3,4-dicarbonsäure-2',5'-dichlor-then-3-yl-amid (II)

Zu 154,4 g (1 M) Thiophen-3,4-dicarbonsäureanhydrid in 2 l Toluol wurden 168,0 g (1 M) 2,5-Dichlor-3-amino-thiophen in 1,5 1 Toluol während 45 min bei Raumtemperatur unter gutem Rühren zugetropft. Man ließ anschließend 2 bis 3 h nachrühren, saugte dann die dick ausgefallenen Festkörper ab, wusch gut mit Toluol nach und trocknete die Festkörper zuerst an der Luft und später im Vakuumtrockenschrank. Man erhielt 313 g (97 %), Fp. 266-268°C (Zersetzung).

b) 1,3-Dichlor-4,5-dihydro-dithieno[3,4-b:3',4'-e]azepin-5,9-dion (III)

10,0 g (31.0 mM) N-(2' ,5'-Dichlor-thien-3-yl)-2-carboxy-thiopen-carbonsäureamid wurden in 350 ml Tetrahydrofuran gelöst und unter gutem Rühren mit 3,5 g (3l,0 mM) N-Hydroxysuccinimid sowie 6,5 g (31,0 mM) N,N-Dicyclohexyl-carbodiimid versetzt. Man ließ das Reaktionsgemisch 2 h bei Raumtemperatur nachrühren, saugte den ausgesfallenen Harnstoff ab, wusch mit wenig Tetrahydrofuran nach und engte das Filtrat zur Trockne ein. Der Rückstand wurde portionsweise in eine Schmelze aus 49,2 g (368 mM) $AlCl_3$ mit 7,4 ml Dimethylformamid bei 80°C Innentemperatur unter gutem Rühren eingetragen. Man hielt die Reaktionsmischung noch 45 min auf 90°C und goß anschließend den Ansatz noch heiß auf Eis, stellte mit HC1 sauer und saugte die hellbraunen Festkörper nach einigem Rühren ab. Nach dem Nachwaschen mit $H_2O$ wurde das Produkt an der Luft getrocknet. Man erhielt 9,1 g (97 %) die nach Umkristallisieren aus Eisessig bei 248-250°C schmolzen.

c) 4,5-Dihydro-dithieno[3,4-b:3',4'-e]azepin-5,9-dion (I)

l9,2 g (63,2 mM) 1,3-Dichlor-4,5-dihydro-dithieno[3,4-b:3',4'-e]azepin-5,9-dion in 600 ml 1-Methyl-pyrrolidon-2 wurden mit 3,0 g Pd/C (10 %) sowie mit l5,0 g (183 mM) fein pulverisiertem Natriumacetat versetzt und im 2,5 l Rollhofer 5 h unter 200 bar Wasserstoffdruck bei 80°C hydriert. Anschließend saugte man ab und wusch mit Pyrrolidon, dann mit $H_2O$. Dann goß man das Filtrat auf 5 l Eiswasser, säuerte mit konzentrierter HCl an, saugte nach 1 h Nachrühren die Festkörper unter gutem Nachwaschen mit $H_2O$ ab und trocknete das Rohprodukt. Man erhielt 12,6 g (85 %), Fp. 267-270°C

<u>Beispiel 2</u>

a) Thiophen-3,4-dicarbonsäure-2',5'-dichlorthen-3-yl-amid (II)

Zu 308,8 g (2.00 M) Thiophen-3,4-dicarbonsäureanhydrid und 416,8 g (2,04 M) 3-Amino-2,5-dichlor-

thiophen-hydrochlorid in 2,2 l Dimethylformamid wurden bei l0°C innerhalb 1 h unter Rühren 202,4 g (2.00 M) Triethylamin getropft. Es wurde noch 2 h bei Raumtemperatur nachgerührt und dann unter Rühren 1 l Wasser zugegeben. Nach einer weiteren Rührzeit von 1 h bei Raumtemperatur wurde das Gemisch unter Rühren auf 5 l Wasser gegossen. Anschließend wurde abgesaugt. Der Filtrierrückstand wurde nacheinander mit Wasser (7 l), Aceton (0,6 l) und Petrolether. (Siedebereich 40-60°C (1 l) gewaschen und im Vakuumtrockenschrank bei 50°C getrocknet. Isoliert wurden 614,5 g (95 %) Thiophen-3,4-bicarbonsäure-2',5'-dichlorthen-3-yl-amid als beige Kristalle, Fp. 250-253°C (mit Zersetzung)

b)Thiophen-3,4-dicarbonsäure-succinimidoester-[N-(2,5-dichlor-then-3-yl)]amid (IV)

Ein Gemisch aus 200 g (0,621 M) Thiopen-3,4-dicarbonsäure-2',5'-dichlorthin-3-yl-amid und 3,2 l Tetrahydrofuran wurde unter Rühren mit 71,7 g (0,623 M) N-Hydroxysuccinimid versetzt. Anschließend wurde unter Rühren innerhalb 15 min eine Lösung von 128,5 g (0.623 M) N,N'-Dicyclohexylcarbodiimid zugetropt. Nach beendeter Zugabe wurde noch 3 h nachgerührt, dann abgesaugt und der Filtrierrückstand mit wenig Tetrahydrofuran gewaschen. Das usprügliche Filtrat wurde nach Vereinigung mit dem Waschtetrahydrofuran unter Rühren auf 6,8 l Petrolether (Siedebereich 40-60°C) gegossen. Die dabei ausgefallenen Kristalle wurden abgesaugt, mit Petrolether (Siedebereich 40-60°C) gewaschen und im Vakuumtrockenschrank bei 50°C getrocknet. Isoliert wurden 251,5 g (97 %) beige Kristalle, Fp 175-184°C.

c) 1,3-Dichlor-4,5-dihydro-dithieno[3,4-b:3'4'-e]azepin-5,9-dion (III)

Zu 798 g (5,98 M) wasserfreiem Aluminiumchlorid wurden unter Rühren innerhalb 15 min 109,5 g (116 ml; 1.50 M) Dimethylformamid gegeben, wobei eine stark exotherme Reaktion eintrat. Anschließend wurden bei 80°C unter Rühren innerhalb 30 min 250 g (0,596 M) Thiopen-3,4-dicarbonsäure-succinimidoester-[N-(2,5-dichlor-thn-3-yl)]amid hinzugefügt. Das Reaktionsgemisch wurde noch 3 h bei 80°C nachgerührt und dann in warmem Zustand unter Rühren auf 4,5 kg Eis und 470 ml (561,2 g; 5,85 M) konzentrierte Salzsäure gegossen. Die dabei ausgefallenen Kristalle wurden abgesaugt, nacheinander mit Wasser (6 l), Aceton (2 x 0,5 l) und Petrolether (Siedebereich 40-60°C) (2 x 0,5 l) gewaschen und im Vakuumtrockenschrank bei 50°C getrocknet. Isoliert wurden 170,8 g (94 %) beige Kristalle, Fp. 236,5-239°C.

d) 4,5-Dihydroditheno[3,4-b:3',4'-e]azepin-5,9-dion (I)

Ein Gemisch aus 125 g (0,411 M) 1,3-Dichlor-4,5-dihydro-dithieno[3,4-b:3',4'-e]azepin-5,9-dion, 125 g (1,982 M) Ammoniumformiat, 50 g Pd/C (5 %ig), 50 g Aktivkohle, 3,125 l Propanol und 165 ml Wasser wurde 3 h unter Rühren bei Rückflußtemperatur gehalten. Anschließend wurden 2,5 l Lösungsmittel bei ca. 100 mbar unter Rühren abdestilliert. Der Rückstand wurde auf 3 l Eiswasser gegossen und die dabei erhalten Suspension unter Rühren mit konzentrierter Salzsäure angesäuert. Es wurde noch 1 h nachge rührt, dann abgesaugt und der Filtrierrückstand zu l,8 l Dimethylformamid gegeben. Nach dem Erwärmen auf l00°C unter Rühren wurde erneut abgesaugt. Der Filterrückstand (Pd/C und Aktivkohle) wurde fünfmal mit je 100 ml heißem Dimethylformamid nachgewaschen. Die ursprüngliche Dimethylformamid-Mutterlauge wurde nach Vereinigung mit dem Wasch-Dimethylformamid noch warm unter Rühren auf 9 l kaltes Wasser gegossen. Der dabei gebildete Niederschlag wurde abgesaugt, mit Wasser gewaschen und im Vakuumtrockenschrank bei 50°C getrocknet. Isoliert wurden 80,6 g (83 %) beige Kristalle, Fp. 268,5-271°C.

Beispiel 3

4,5-Dihydro-ditheno[3,4-b:3',4'-e]azepin-5,9-dion (I)

Ein Gemisch aus 5,0 g (0,0164 M) 1,3-Dichlor-4,5-dihydro-dithieno[3,4-b:3',4'-e]azapin-5,9-dion, 4 g Pd/C (5 %ig), 6,3 g (0,034 M) Tributylamin und 300 ml Propanol wurde in einer Wasserstoffatomosphäre bei 80°C gerührt. Nach beendigter Wasserstoffaufnahme (5 h) wurde heiß filtriert und das Filtrat auf Eiswasser gegossen. Die dabei erhaltene Suspension wurde mit konzentrierter Salzsäure angesäuert. Anschließend wurde kurz nachgerührt und danach abgesaugt. Der Filtrierrückstand aus der Filtration des rohen Reaktionsgemisches wurde mehrmals mit heißem Dimethylformamid gewaschen. Das Wasch-Dimethylformamid wurde auf Eisewasser gegossen und der dabei gebildete Niederschlag abgesaugt. Der so erhaltene Festkörper wurde mit dem aus dem Propanol-Filtrat erhaltenen Festprodukt vereinigt. Das gesamte Festprodukt wurde mit Wasser gewaschen und dann im Vakuumtrockenschrank bei 50°C getrocknet. Isoliert wurden 2,9 g (75 %) beige Kristalle, Fp. 268-271°C.

## Patentansprüche

1. Verfahren zur Herstellung von 4,5-Dihydro-dithieno[3,4-b:3',4'-e]azepin-5,9-dion, dadurch gekennzeichnet, daß man Thiophen-3,4-dicarbonsäure-2',5'-dichlor-then-3-yl-amid durch Behandlung mit

N-Hydroxysuccinimid in Gegenwart eines Moläquivalents eines Kondensationsmittels in den Ester der Formel IV

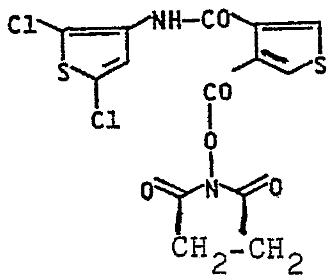

IV

überführt, diesen nach Friedel-Crafts cyclisiert und danach entchloriert.

2. 1,3-Dichlor-4,5-dihydro-dithieno[3,4-b:3',4'-e]azepin-5,9-dion.

3. Verbindung der Formel IV gemäß Anspruch 1.

## Claims

1. A process for the preparation of 4,5-dihydrodithieno[3,4-b:3',4'-e]azepine-5,9-dione, wherein thiophene-3,4-dicarboxylic acid 2',5'-dichlorothien-3-ylamide is treated with N-hydroxysuccinimide in the presence of one mole equivalent of a condensing agent to give an ester of the formula IV

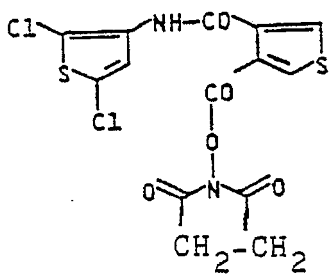

IV

the latter is subjected to a Friedel-Crafts cyclization reaction and the product is then dechlorinated.

2. 1,3-Dichloro-4,5-dihydrodithieno[3,4-b:3',4'-e]azepine-5,9-dione.

3. A compound of the formula IV according to claim 1.

## Revendications

1. Procédé de préparation de 4,5-dihydro-dithienol[3,4-b:3',4'-e]-azepin-5,9-dione, caractérisé par le fait qu'un transforme du 2',5'-dichloro-then-3-ylamide d'acide thiophen-3,4-dicarboxylique, par traitement avec du N-hydroxysuccinimide, en présence d'un équivalent molaire d'un agent de condensation, en l'ester de formule IV,

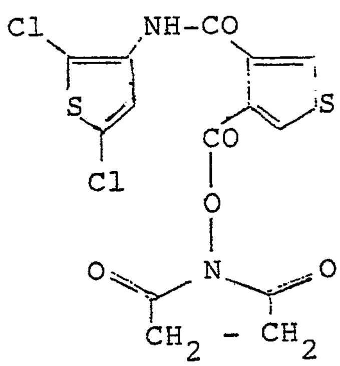

IV

on cyclise celui-ci selon Friedel-Crafts, puis on sépare le chlore.

2. 1,3-dichlor-4,5-dihydro-dithienol[3,4-b:3',4'-e]azepin-5,9-dione.

3. Composé de formule IV selon la revendication 1.